# EUROPÄISCHE PATENTSCHRIFT

(11) **EP 0 634 157 B1**
(45) Veröffentlichungstag und Bekanntmachung des Hinweises auf die Patenterteilung: **31.01.1996**
(21) Anmeldenummer: 94110501.7
(22) Anmeldetag: 06.07.1994
(51) Int. Cl.: A61F 2/44

(54) **Bandscheibenteilersatz**
Partial intervertebral disc prosthesis
Prothèse partielle pour disques intervertébraux

(30) Priorität: 15.07.1993 DE 4323595
(43) Veröffentlichungstag der Anmeldung: 18.01.1995
(73) Patentinhaber: ESKA medical GmbH & Co., D-23556 Lübeck (DE)
(72) Erfinder: Grundei, Hans, Dr., D-23558 Lübeck (DE)
(74) Vertreter: Dr. Fuchs, Dr. Luderschmidt Dr. Mehler, Dipl.-Ing Weiss Patentanwälte

(56) Entgegenhaltungen:
- EP-A- 0 042 271
- EP-A- 0 260 044
- EP-A- 0 307 241
- EP-A- 0 317 972

## Beschreibung

Die vorliegende Erfindung betrifft einen Bandscheibenteilersatz, welcher als Entlastungsteil in lädierten Bandscheiben zum Einsatz kommt. Bei lädierten Bandscheiben besteht die Gefahr, daß zwei benachbarte Wirbel aufeinander reiben. Selbstverständlich kann es auch zu dem weit verbreiteten Bandscheibenvorfall kommen, bei dem Druck auf den Spinalkanal ausgeübt wird, infolge dessen es im schlimmsten Falle zu einer Lähmung kommen kann.

Künstliche Bandscheibenendoptrothesen sind in vielfältiger Ausgestaltung bekannt. Beispielhaft sei hier jene Bandscheibenendoprothese gemäß der DE 42 13 771 C1 der Anmelderin genannt. Die darin offenbarte Bandscheibenendoprothese ist eine Vollendoprothese, in dem Sinne, daß die beschädigte Bandscheibe vollständig entfernt und ersetzt wird durch die Endoprothese.

Die Applikation dieser künstlichen Bandscheibe ist nicht in allen Fällen angezeigt und vonnöten. In vielen, wenn nicht gar in den meisten Fällen würde eine lokale Ausbesserung der lädierten Bandscheibe ausreichen, um negative Konsequenzen aus der Läsion zu vermeiden. Derartige, nur lokal wirkende Implantate sind zum Beispiel aus der EP-A-307241 bekannt, wobei die Bandscheibe teilweise entfernt wird und die zwei benachtbarten Wirbel in ihrer Lage zueinander fixiert werden.

Es ist daher die Aufgabe der vorliegenden Erfindung, ein Implantat anzugeben, welches bei lädierten Bandscheiben zu einer spürbaren Entlastung führt.

Gelöst wird die Aufgabe durch einen Bandscheibenteilersatz gemäß dem Anspruch 1. Vorteilhafte Ausgestaltungen ergeben sich aus den Unteransprüchen.

Demgemäß wird als Implantat ein Bandscheibenteilersatz vorgeschlagen, der als Entlastungsteil wirkt. Der Bandscheibenteilersatz besteht aus einer distal abgerundeten Hülse aus elastischem Material und einem metallischen Körper mit einem Zapfen, der an einer Abschlußplatte angebracht ist. Die Hülse weist in ihrem Inneren einen Aufnahmeraum für den Zapfen auf. Wird der Zapfen in die Hülse eingeführt, so liegt schließlich die Abschlußplatte an der Stirnseite der Höhle an und schließt mit dieser im wesentlichen bündig ab. Die Hülse ist also armiert durch den metallischen Zapfen.

Der Einsatz dieses Teilersatzes kann folgendermaßen ablaufen: in der Regel werden pro Bandscheibe zwei Implantate eingesetzt. Etwa in Richtung der Querfortsätze der Wirbelkörper wird jeweils eine Bohrung in die beschädigte Bandscheibe eingebracht. Jeweils ein Bandscheibenteilersatz wird daraufhin in die Bohrung gesetzt.

Vorzugsweise besteht die Hülse aus körperverträglichem Silikon. Der metallische Teil aus Abschlußplatte und Zapfen hingegen besteht aus körperverträglichem Metall.

Zur Erhöhung der Verbundfestigkeit zwischen dem Silikon der Hülse und dem Metall des Zapfens ist dieser vorzugsweise mit einem oberflächenvergrößernden Profil versehen.

Nachfolgend wird eine besonders bevorzugte Ausführungsform des Bandscheibenteilersatzes beschrieben. Hier weist die Abschlußplatte einen umlaufenden Bund auf. Dieser Bund faßt die mit einem Absatz am proximalen Ende versehene Hülse ein. Der durchmesservermindernde Absatz der Hülse ist so ausgebildet, daß der Bund dennoch bündig mit der Hülse abschließt. Außen ist der umlaufende Bund mit einer dreidimensionalen offenmaschigen Raumnetzstruktur versehen, wie sie beispielsweise bekannt ist aus der DE 41 06 971 C1, wobei diese Struktur im vorliegenden Falle nicht dazu dient, daß Knochentrapekel in sie hineinwachsen sollen, sondern vielmehr Bindegewebe zur Stabilisierung und Fixation des Bandscheibenteilersatzes.

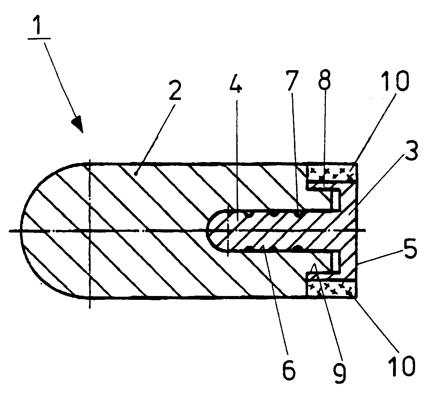

Die Erfindung wird anhand der einzigen Zeichnungsfigur näher erläutert. Diese zeigt eine Ausführungsform des erfindungsgemäßen Bandscheibenteilersatzes im Schnitt.

Der Bandscheibenteilersatz 1 besteht aus einer distal abgerundeten Hülse 2. Diese weist in ihrem Inneren einen Aufnahmeraum 6 auf. Der Aufnahmeraum ist in einfacher Weise als zentrische Bohrung in der Hülse 2 ausgeführt. In den Aufnahmeraum 6 eingeführt dargestellt ist der Zapfen 4 des metallischen Teils des Implantats. Dieser ist angeformt an eine Abschlußplatte 5. Die Längenverhältnisse von Zapfen 4 und Aufnahmeraum sind so gewählt, daß die Abschlußplatte des metallischen Körpers 3 im wesentlichen an der Stirnseite der Hülse 2 anliegt.

Im abgebildeten Ausführungsbeispiel verfügt der Zapfen 4 über ein oberflächenvergrößerndes Profil 7, wodurch die Verbundfestigkeit zwischen dem Hülsenmaterial und dem metallenen Zapfen erhöht wird.

Proximal weist die Hülse 2 einen durchmesserverringernden Absatz 9 auf. Dieser Absatz 9 korrespondiert in seiner Tiefe mit der Höhe des an der Abschlußplatte 5 angeformten und umlaufenden Bundes 8 mit darauf vorgesehener dreidimensionaler offenmaschiger Raumnetzstruktur 10.

Die Raumnetzstruktur 10 ist vorliegend dafür vorgesehen, daß hier Bindegewebe einwachsen kann.

## Patentansprüche

1. Bandscheibenteilersatz (1) zum Einbringen in eine Bandscheibe als Entlastungsteil, bestehend aus einer distal abgerundeten Hülse (2) aus elastischem Material und einem metallischen Körper (3) mit einem Zapfen (4), der an einer Abschlußplatte (5) angebracht ist, wobei die Hülse (2) in ihrem Inneren einen Aufnahmeraum (6) für den Zapfen (4) aufweist, dergestalt, daß die Abschlußplatte (5) bei in die Hülse (2) gesetztem Zapfen (4) im wesentlichen an der Stirnseite der Hülse (2) anliegt und im wesentlichen bündig mit dieser abschließt.

2. Bandscheibenteilersatz nach Anspruch 1, bei der die Hülse (2) aus Silikon besteht.

3. Bandscheibenteilersatz nach Anspruch 1 oder 2, bei der Zapfen (4) mit einem oberflächenvergrößernden Profil (7) versehen ist.

4. Bandscheibenteilersatz nach einem der Ansprüche 1 bis 3, bei der die Abschlußplatte (5) einen umlaufenden Bund (8) aufweist, der die mit einem Absatz (9) am proximalen Ende versehene Hülse (2) einfaßt und außen eine dreidimensionale offenmaschige Raumnetzstruktur (10) aufweist.

## Claims

1. Partial intervertebral disc prosthesis (1) for introduction into an intervertebral disc as a relieving part, consisting of a distally rounded sleeve (2) made from resilient material and a metal body (3) having a pin (4) which is attached to a closure plate (5), wherein the interior of sleeve (2) has a receiving space (6) for pin (4) such that closure plate (5) rests essentially against the end-face side of sleeve (2) when pin (4) is placed in sleeve (2) and closes essentially flush with the latter.

2. Partial intervertebral disc prosthesis according to claim 1, in which sleeve (2) consists of silicone.

3. Partial intervertebral disc prosthesis according to claim 1 or 2, in which pin (4) is provided with a surface-enlarging profile (7).

4. Partial intervertebral disc prosthesis according to one of claims 1 to 3, in which closure plate (5) has an annular collar (8), which encloses sleeve (2) provided with a recess (9) on the proximal end and externally has a three-dimensional open-meshed spatial reticular structure (10).

## Revendications

1. Prothèse partielle (1) de disque intervertébral, destinée à être insérée dans un disque intervertébral, en tant qu'élément de détente, constitué par une douille (2) arrondie à son extrémité distale et réalisée en un matériau élastique et par un corps métallique (3) possédant une tige (4), qui est montée sur une plaque terminale (5), la douille (2) possédant, en son intérieur, un espace de logement (6) pour la tige (4), de telle sorte que la plaque terminale (5), lorsque la tige (4) est insérée dans la douille (2), s'applique essentiellement contre la face frontale de la douille (2), et se termine sensiblement de niveau avec cette douille.

2. Prothèse partielle de disque intervertébral selon la revendication 1, dans laquelle la douille (2) est réalisée en silicone.

3. Prothèse partielle de disque intervertébral selon la revendication 1 ou 2, dans laquelle la tige (4) possède un profil (7) qui augmente la surface.

4. Prothèse partielle de disque intervertébral selon l'une des revendications 1 à 3, dans laquelle la plaque terminale (5) possède un collet circonférentiel (8), qui enserre la douille (2) comportant un épaulement (9) sur son extrémité proximale, et possède, sur le côté extérieur, une structure réticulés tridimensionnelle (10) à mailles ouvertes.
